# EUROPEAN PATENT APPLICATION

(11) **EP 3 852 116 A1**
(43) Date of publication of application: **21.07.2021**
(21) Application number: 20152483.2
(22) Date of filing: 17.01.2020
(51) Int. Cl.: G16H 15/00, G16H 80/00

(54) **APPARATUS FOR ANALYZING A RECORD OF A VERBAL COMMUNICATION**

(71) Applicant: Koninklijke Philips N.V., 5656 AG Eindhoven (NL)
(72) Inventor: LAUTE, Niels, 5656 AE Eindhoven (NL); GEURTS, Lucas Jacobus Franciscus, 5656 AE Eindhoven (NL)
(74) Representative: Philips Intellectual Property & Standards

(57) **Abstract**

There is provided an apparatus (100) for generating a record of a verbal communication. The apparatus (100) comprises one or more processors (102) configured to analyze a verbal communication to identify one or more clinical statements made by one or more participants of the verbal communication and, for each of the one or more identified clinical statements, identify the participant that made the clinical statement and the expertise of the participant that made the clinical statement and assign a score to the clinical statement based on a content of the clinical statement and the expertise of the participant that made the clinical statement. The one or more processors (102) are configured to generate a record of the verbal communication. The generated record comprises the one or more identified clinical statements and the respective scores assigned to the one or more identified clinical statements.

## Description

### FIELD OF THE INVENTION

The disclosure relates to an apparatus and a computer-implemented method for generating a record of a verbal communication.

### BACKGROUND OF THE INVENTION

There are various situations in which verbal communications between participants can be critical to future events. This is particularly the case in the medical field where clinical statements made by one or more of the participants of the verbal communication can be critical to managing the health and wellbeing of a patient. For example, meetings between medical personnel may make available a large amount of information on individual patients and often result in a decision being taken on the treatment, or follow-up treatment, for the patients.

An example of meetings in which this is often the case are multi-disciplinary tumor board meetings. In such tumor board meetings, many cancer patient cases are discussed in short time. In an average tumor board, around 10 attendees are part of the decision-making committee, each with their own specialty. The chairperson handles the case list (which can range from between 20 to 40 cases per session depending on its length), while the average discussion time per patient is around 3 to 5 minutes. The session requires the chairperson to explain to the team who the patient is to give them a sense of the profile of the patient. Then, a structured process is followed to determine what the best treatment will be for the patient. These sessions range from relatively simple patient cases to very complex patient cases that require more discussion and planning.

Generally, in meetings between medical personnel, notetaking is mostly undertaken by a medical student or trainee that is attending the meeting. These notes are often entered into information systems (e.g. an electronic medical record, EMR) as free text, which is unstructured, open for interpretation, and can also sometimes be missing important and relevant details. There is thus no guarantee that the notes are reliable. US 2019/0028520 discloses a system that relates to the automation of tasks using machine learning, which includes the generation of corporate minute documents in compliance with legal and organizational requirements. The system determines a level of confidence that a particular component of text stream data is relevant to a final summary document. However, the system still suffers from the same disadvantages mentioned earlier and thus there is no guarantee that the final summary document produced by this system is reliable.

### SUMMARY OF THE INVENTION

As noted above, the limitations with existing techniques for recording information is that the information is generally unstructured, open for interpretation, and can be missing important and relevant details. There is thus no guarantee that the notes are reliable, which can be particularly problematic in the clinical domain. It would therefore be valuable to have an improvement aimed at addressing these limitations.

Therefore, according to a first aspect, there is provided an apparatus for generating a record of a verbal communication. The apparatus comprises one or more processors configured to analyze a verbal communication to identify one or more clinical statements made by one or more participants of the verbal communication. The one or more processors are configured to, for each of the one or more identified clinical statements, identify the participant that made the clinical statement and the expertise of the participant that made the clinical statement and assign a score to the clinical statement based on a content of the clinical statement and the expertise of the participant that made the clinical statement. The one or more processors are configured to generate a record of the verbal communication. The generated record comprises the one or more identified clinical statements and the respective scores assigned to the one or more identified clinical statements.

In some embodiments, the generated record may comprise the one or more identified clinical statements organized according to the respective scores assigned to the one or more identified clinical statements.

In some embodiments, the one or more processors may be configured to assign the score to the clinical statement based on the content of the clinical statement and whether the participant that made the clinical statement has expertise in a field to which the content of the clinical statement relates. In some embodiments, the higher the expertise of the participant that made the clinical statement, the higher the score that may be assigned to the clinical statement.

In some embodiments, the one or more processors may be configured to, for each of the one or more identified clinical statements, compare the content of the clinical statement to a content of one or more reference clinical statements stored in at least one memory and/or medical data stored in at least one memory. In some embodiments, the medical data stored in the at least one memory may comprise medical data for a subject to which the verbal communication relates and/or medical data associated with clinical guidelines. In some embodiments, the one or more processors may be configured to assign the score to the clinical statement based on the content of the clinical statement, the expertise of the participant that made the clinical statement, and an output of the comparison.

In some embodiments, the one or more processors may be configured to determine whether there is a discrepancy between the content of two or more identified clinical statements and, if there is determined to be a discrepancy between the content of two or more identified clinical statements, generate an indication of the discrepancy. In some embodiments, the generated record of the verbal communication may comprise the generated indication of the discrepancy.

In some embodiments, the one or more processors may be configured to control at least one user interface to render the generated record of the verbal communication. In some embodiments, the generated record may comprise an indication of the participant that made the one or more identified clinical statements.

In some embodiments, the one or more processors may be configured to compare the content of the one or more identified clinical statements to an indication of the content that the verbal communication is to cover, determine whether any content that the verbal communication is to cover is missing from the content of the one or more identified clinical statements, and if content is determined to be missing, generate an indication that the content is missing.

In some embodiments, the verbal communication may comprise a real-time verbal communication and/or a recorded verbal communication.

According to a second aspect, there is provided a method for generating a record of a verbal communication. The method comprises analyzing a verbal communication to identify one or more clinical statements made by one or more participants of the verbal communication. The method comprises, for each of the one or more identified clinical statements, identifying the participant that made the clinical statement and the expertise of the participant that made the clinical statement and assigning a score to the clinical statement based on a content of the clinical statement and the expertise of the participant that made the clinical statement. The method comprises generating a record of the verbal communication. The generated record comprises the one or more identified clinical statements and the respective scores assigned to the one or more identified clinical statements.

According to a third aspect, there is provided a computer program product comprising a computer readable medium. The computer readable medium has a computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, the record of the verbal communication that is generated comprises one or more identified clinical statements and respective scores that have been assigned to those one or more identified clinical statements. The score assigned to each of the one or more identified clinical statements provides context to the clinical statement, since the score is assigned based on a content of the clinical statement and also the expertise of the participant that made the clinical statement. In particular, the score can provide an indication of the reliability of the clinical statement. There is thus provided an improved technique for generating a record of a verbal communication.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an apparatus according to an embodiment; and
Fig. 2 is a flow chart illustrating a method according to an embodiment.

### DETAILED DESCRIPTION OF EMBODIMENTS

As noted above, there is provided herein an improved technique for generating a record of a verbal communication. Herein, a verbal communication can, for example, be a real-time verbal communication and/or a recorded verbal communication.

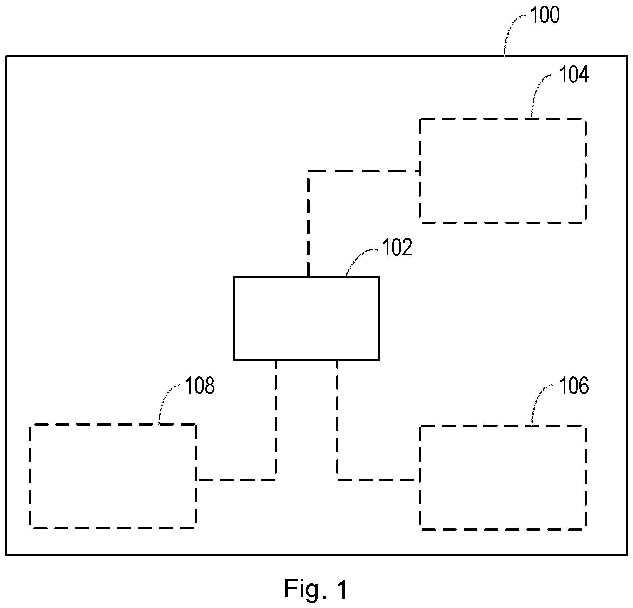

Fig. 1 illustrates an apparatus 100 for generating a record of a verbal communication according to an embodiment. As illustrated in Fig. 1, the apparatus 100 comprises one or more processors 102.

The one or more processors 102 can be implemented in numerous ways, with software and/or hardware, to perform the various functions described herein. In particular implementations, the one or more processors 102 can comprise a plurality of software and/or hardware modules, each configured to perform, or that are for performing, individual or multiple steps of the method described herein. The one or more processors 102 may comprise, for example, one or more microprocessors, one or more multi-core processors and/or one or more digital signal processors (DSPs), one or more processing units, and/or one or more controllers (e.g. one or more microcontrollers) that may be configured or programmed (e.g. using software or computer program code) to perform the various functions described herein. The one or more processors 102 may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

Briefly, one or more processors 102 of the apparatus 100 are configured to, analyze a verbal communication to identify one or more clinical statements (e.g. remarks, hypotheses, conclusions, etc.) made by one or more participants of the verbal communication. Also, the one or more processors 102 are configured to, for each of the one or more identified clinical statements, identify the participant that made the clinical statement and the expertise of the participant that made the clinical statement and assign a score to the clinical statement based on a content of the clinical statement and the expertise of the participant that made the clinical statement. Further, the one or more processors 102 of the apparatus 100 are configured to generate a record of the verbal communication. The generated record comprises the one or more identified clinical statements and the respective scores assigned to the one or more identified clinical statements.

Herein, the one or more participants may, for example, comprise one or more human participants and/or one or more artificial intelligence (AI) participants. In some cases, one or more human participants may include one or more medical professionals, e.g. one or more doctors, one or more surgeons, one or more nurses, and/or any other medical professionals. The expertise of a human participant can, for example, comprise an amount of skill and/or knowledge, such as an amount of skill and/or knowledge in a particular field. In some cases, one or more AI participants may include one or more computer-controlled robots, and/or any other AI participants. The expertise of an AI participant can, for example, comprise an amount of training data used to train the AI participant to make clinical statements. The participants of the verbal communication can, for example, be attendees of a meeting. The meeting may, for example, be a multi-disciplinary (MD) meeting, such as a multi-disciplinary tumor (MDT) board meeting. In some embodiments involving all AI participants, the technique described herein can be completely automated.

In some embodiments, all of the participants of the verbal communication may be at the same location. In other embodiments, at least one of the participants of the verbal communication may be located remotely from at least one other of the participants of the verbal communication. For example, at least one of the participants of the verbal communication may be located at a different hospital (e.g. a hospital that is specialized in certain cases) to at least one other of the participants of the verbal communication.

As illustrated in Fig. 1, in some embodiments, the apparatus 100 may comprise at least one memory 104. Alternatively or in addition, in some embodiments, at least one memory 104 may be external to (e.g. separate to or remote from) the apparatus 100. For example, another apparatus may comprise at least one memory 104 according to some embodiments. In some embodiments, a hospital database may comprise at least one memory 104, at least one memory 104 may be a cloud computing resource, or similar. The one or more processors 102 of the apparatus 100 may be configured to communicate with and/or connect to at least one memory 104. The at least one memory 104 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 104 can be configured to store program code that can be executed by the one or more processors 102 of the apparatus 100 to cause the apparatus 100 to operate in the manner described herein.

Alternatively or in addition, at least one memory 104 can be configured to store information required by or resulting from the method described herein. For example, at least one memory 104 may be configured to store one or more identified clinical statements, an indication of the identified participant that made one or more clinical statements, an indication of the expertise of the participant that made one or more clinical statements, the score assigned to one or more clinical statements, the generated record of the verbal communication, one or more reference clinical statements, and/or medical data. At least one memory 104 may also be configured to store any other information, or any combination of information, required by or resulting from the method described herein. The one or more processors 102 of the apparatus 100 can be configured to control at least one memory 104 to store information required by or resulting from the method described herein.

As illustrated in Fig. 1, in some embodiments, the apparatus 100 may comprise at least one user interface 106. Alternatively or in addition, in some embodiments, at least one user interface 106 may be external to (e.g. separate to or remote from) the apparatus 100. The one or more processors 102 of the apparatus 100 may be configured to communicate with and/or connect to at least one user interface 106. In some embodiments, the one or more processors 102 of the apparatus 100 can be configured to control at least one user interface 106 to operate in the manner described herein. For example, in some embodiments, the one or more processors 102 of the apparatus 100 may be configured to control at least one user interface 106 to render the generated record of the verbal communication.

A user interface 106 can be configured to render (or output, display, or provide) information required by or resulting from the method described herein. For example, in some embodiments, one or more user interfaces 106 may be configured to render (or output, display, or provide) one or more identified clinical statements, an indication of the identified participant that made one or more clinical statements, an indication of the expertise of the participant that made one or more clinical statements, the score assigned to one or more clinical statements, the generated record of the verbal communication, and/or any other information, or any combination of information, required by or resulting from the method described herein. Alternatively or in addition, one or more user interfaces 106 can be configured to receive a user input. For example, one or more user interfaces 106 may allow a user (e.g. the subject or another user) to manually enter information or instructions, interact with and/or control the apparatus 100. Thus, one or more user interfaces 106 may be any one or more user interfaces that enable the rendering (or outputting, displaying, or providing) of information and/or enables a user to provide a user input.

For example, one or more user interfaces 106 may comprise one or more switches, one or more buttons, a keypad, a keyboard, a mouse, a display or display screen, a graphical user interface (GUI) such as a touch screen, an application (e.g. on a smart device such as a tablet, a smart phone, or any other smart device), or any other visual component, one or more speakers, one or more microphones or any other audio component, one or more lights (e.g. one or more light emitting diodes, LEDs), a component for providing tactile or haptic feedback (e.g. a vibration function, or any other tactile feedback component), an augmented reality device (e.g. augmented reality glasses, or any other augmented reality device), a smart device (e.g. a smart mirror, a tablet, a smart phone, a smart watch, or any other smart device), or any other user interface, or combination of user interfaces. In some embodiments, one or more user interfaces that are controlled to render information may be the same as one or more user interfaces that enable the user to provide a user input.

As illustrated in Fig. 1, in some embodiments, the apparatus 100 may comprise at least one communications interface (or communications circuitry) 108. Alternatively or in addition, in some embodiments, at least one communications interface 108 may be external to (e.g. separate to or remote from) the apparatus 100. A communications interface 108 can be for enabling the apparatus 100, or components of the apparatus 100 (e.g. the one or more processors 102, one or more memories 104, one or more user interfaces 106 and/or any other components of the apparatus 100), to communicate with and/or connect to each other and/or one or more other components. For example, one or more communications interfaces 108 can be for enabling the one or more processors 102 of the apparatus 100 to communicate with and/or connect to one or more memories 104, one or more user interfaces 106 and/or any other components of the apparatus 100.

A communications interface 108 may enable the apparatus 100, or components of the apparatus 100, to communicate and/or connect in any suitable way. For example, one or more communications interfaces 108 may enable the apparatus 100, or components of the apparatus 100, to communicate and/or connect wirelessly, via a wired connection, or via any other communication (or data transfer) mechanism. In some wireless embodiments, for example, one or more communications interfaces 108 may enable the apparatus 100, or components of the apparatus 100, to use radio frequency (RF), Bluetooth, or any other wireless communication technology to communicate and/or connect.

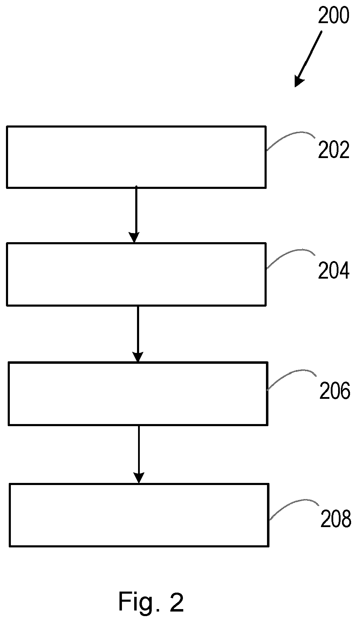

Fig. 2 illustrates a method 200 for generating a record of a verbal communication according to an embodiment. More specifically, Fig. 2 illustrates a method 200 of operating the apparatus 100 described earlier with reference to Fig. 1 for generating a record of a verbal communication. The method 200 illustrated in Fig. 2 is a computer-implemented method. As described earlier, the apparatus 100 described earlier with reference to Fig. 1 comprises the one or more processors 102. The method 200 illustrated in Fig. 2 can generally be performed by or under the control of the one or more processors 102 of the apparatus 100 described earlier with reference to Fig. 1. The method can be at least partially automated. That is, at least part of or all of the method can be performed automatically.

With reference to Fig. 2, at block 202, a verbal communication is analyzed to identify one or more clinical statements (e.g. remarks, hypotheses, conclusions, etc.) made by one or more participants of the verbal communication. More specifically, the one or more processors 102 of the apparatus 100 analyze a verbal communication to identify one or more clinical statements made by one or more participants of the verbal communication.

At block 204, for each of the one or more identified clinical statements, the participant that made the clinical statement and the expertise of the participant that made the clinical statement is identified. More specifically, the one or more processors 102 of the apparatus 100 identify the participant that made each of the clinical statements and the expertise of the participant that made each of the clinical statements.

In some embodiments, a participant that made a clinical statement may be identified from an audio signal indicative of sound in the environment of the participants of the verbal communication, such as through voice recognition. In these embodiments, the one or more processors 102 of the apparatus 100 may be configured to acquire, from an audio sensor (e.g. a microphone), the audio signal indicative of sound in the environment of the participants of the verbal communication. Then, the audio signal acquired at the time a participant made a clinical statement may be analyzed (e.g. using a voice recognition technique) to identify the participant that made the clinical statement.

In some embodiments, the one or more processors 102 of the apparatus 100 may be configured to analyze the audio signal acquired at the time the participant made the clinical statement by being configured to compare the audio signal acquired at the time the participant made the clinical statement to a plurality of sample audio signals stored for each participant of the verbal communication in at least one memory 104 (e.g. at least one memory of the apparatus 100 and/or at least one memory external to the apparatus 100). For example, one or more voice characteristics may be extracted from the audio signal acquired at the time the participant made the clinical statement and compared to one or more corresponding voice characteristics of the plurality of sample audio signals stored for each participant of the verbal communication in at least one memory 104 (e.g. at least one memory of the apparatus 100 and/or at least one memory external to the apparatus 100).

The participant that made the clinical statement may be identified as the participant for which the stored sample audio signal most closely matches (i.e. differs the least from) the acquired audio signal, such as the participant for which the one or more voice characteristics of the stored sample audio signal most closely match (i.e. differ the least from) the one or more corresponding voice characteristics of the audio signal acquired at the time the participant made the clinical statement. The one or more processors 102 of the apparatus 100 can be trained to recognize the voices of each of the participants of the verbal communication according to some embodiments.

In some embodiments, identifying the participant that made the clinical statement may also involve a unique identifier assigned to the participant, such as a national provider identifier (NPI) or any other unique identifier. For example, at least one memory 104 (e.g. at least one memory of the apparatus 100 and/or at least one memory external to the apparatus 100) may be configured to store a unique identifier for each participant of the verbal communication together with the corresponding audio signal of the voice of each participant. Thus, for each participant of the verbal communication, the audio signal of the voice of the participant can be mapped to a unique identifier that identifies the participant. In these embodiments, the one or more processors 102 of the apparatus 100 may be configured to compare an audio signal acquired at the time a participant made a clinical statement to the stored audio signals and identify the participant that made the clinical statement from the identifier that is stored with an audio signal that most closely matches (i.e. differs the least from) the acquired audio signal, such as the identifier that is stored with an audio signal for which one or more voice characteristics most closely match (i.e. differ the least from) one or more corresponding voice characteristics of the acquired audio signal.

In some embodiments, the one or more processors 102 may use a data model comprising data indicative of the expertise of the participants of the verbal communication (e.g. the attendees of a meeting in which the verbal communication is taking place) to identify the expertise of the participant that made each of the clinical statements. For example, for each participant, the data model may comprise data about the organizational structure of the participants of the verbal communication, such as an indication of a person to which the participant reports, an indication of a clinical domain of the participant, an indication of one or more specialties of the participant, and so on. The data model may be referred to as an organizational model. In some embodiments, the expertise of a participant that made a clinical statement may be identified by comparing the identity (e.g. the unique identifier, such as the NPI) of the identified participant to a plurality of identities (e.g. unique identities, such as NPIs) stored for each participant of the verbal communication in at least one memory 104 (e.g. at least one memory of the apparatus 100 and/or at least one memory external to the apparatus 100) with an indication of their expertise.

The indication of expertise of a participant may be in the form of a level according to some embodiments and thus may be referred to as an expertise level. In some embodiments, the stored indication of expertise of each participant of the verbal communication can be based on previous cases and/or can be (e.g. continuously) updated. In an example, an expertise level (e.g. in a specific field) of a participant may be set based on one or more cases to which the participant contributed in the past (e.g. based on types of clinical statements) and the outcome of the one or more cases. In some embodiments, the academic background and/or experience (e.g. months of working) in a specific field may be taken into account in the setting of an expertise level of a participant.

Although not illustrated in Fig. 2, in some embodiments, any one or more of an emotional state, a stress level and a tiredness level of the participant that made the clinical statement may be detected. More specifically, the one or more processors 102 of the apparatus 100 may be configured to detect any one or more of the emotional state, the stress level and the tiredness level of the participant that made the clinical statement. For example, the emotional state of the participant that made the clinical statement may be detected based on the speech of that participant and/or by other means. Similarly, for example, the stress level and/or the tiredness level of the participant that made the clinical statement may be detected based on the voice of that participant (e.g. recorded with a microphone), an image of that participant (e.g. recorded with a camera) and/or by other means (e.g. wearables).

Thus, in this way, for each of the one or more identified clinical statements, the participant that made the clinical statement and the expertise of the participant that made the clinical statement can be identified. Although examples have been provided for the way in which the participant that made the clinical statement and the expertise of that participant may be identified, a person skilled in the art will be aware of other ways that can be used.

Returning back to Fig. 2, at block 206, for each of the one or more identified clinical statements, a score is assigned to the clinical statement based on a content of the clinical statement and the expertise of the participant that made the clinical statement. More specifically, the one or more processors 102 of the apparatus 100 assign the score to each of the clinical statements. In some embodiments, the expertise of the participant that made the clinical statement may be whether the participant that made the clinical statement has expertise in a field to which the content of the clinical statement relates. As the score is assigned to the clinical statement based on the content of the clinical statement and the expertise of the participant that made the clinical statement, the score can be indicative of background information, e.g. on the role of the participant, such as a specialist (e.g. radiologist specialized in "X", with "Y" years of experience) and/or a track record of the participant in previous (e.g. similar) cases (e.g. X number of previous cases were correctly diagnosed by this participant). This can provide an indication of whether a clinical statement is in line with the expertise of the participant that made the clinical statement, for example, whether it is in line with the clinical expertise domain of the participant and/or the experience level that the participant has on a topic that is the subject of the verbal communication.

In some embodiments, for example, the higher the expertise of the participant that made the clinical statement, the higher the score that is assigned to the clinical statement. In some embodiments, if a participant makes a clinical statement that is beyond the expertise (e.g. outside the domain or experience) of the participant, a lower score is assigned to the clinical statement than if the participant makes a clinical statement that is within the expertise (e.g. inside the domain or experience) of the participant. For example, if a radiologist makes a clinical statement on a genomics topic, a lower score is assigned to the clinical statement than if the radiologist makes a clinical statement on image data. The score may be a percentage in some embodiments. In some embodiments, a weighting factor may be used to calculate the score. In some embodiments, the score may be calculated based on previous cases (e.g. and the history thereof) and external publications and/or presentations. For example, this information can be used to create a historical profile (e.g. about domain knowledge, etc.) that can then be used as a weighing factor to calculate the score.

In embodiments where an emotional state, a stress level and/or a tiredness level of the participant that made the clinical statement is detected, for each of the one or more identified clinical statements, the score may be assigned to the clinical statement based on the content of the clinical statement, the expertise of the participant that made the clinical statement and also any one or more of the detected emotional state, stress level and tiredness level of the participant that made the clinical statement. In some embodiments, for example, the more stable the emotional state of the participant that made the clinical statement, the higher the score that is assigned to the clinical statement. For example, if a participant is detected to be in a calm emotional state, a higher score is assigned to the clinical statement than if the participant that made the clinical statement is in an agitated emotional state. Similarly, for example, the lower the stress level and/or tiredness level of the participant that made the clinical statement, the higher the score that is assigned to the clinical statement.

Returning back to Fig. 2, at block 208, a record of the verbal communication is generated. More specifically, the one or more processors 102 of the apparatus 100 generate a record of the verbal communication. The generated record comprises the one or more identified clinical statements and the respective scores assigned to the one or more identified clinical statements. Thus, the one or more identified clinical statements can be annotated with the respective scores assigned to them. The record of the verbal communication can be in the form of a transcript of the one or more identified clinical statements. In this way, a (e.g. an automated) note taking system or transcription service is provided in which one or more clinical statements of the participants of the verbal communication are transcribed. The record of the verbal communication can be generated in real time according to some embodiments. The record of the verbal communication may comprise one or more labels e.g. that identify intents and/or facts.

In some embodiments, the one or more identified clinical statements can be structured according to a pre-defined structure and/or protocol. In some embodiments, the generated record may comprise the one or more identified clinical statements organized according to the respective scores assigned to the one or more identified clinical statements. For example, one or more identified clinical statements may be selected and/or prioritized according to the respective scores assigned to the one or more identified clinical statements. Thus, in some embodiments, the record of the verbal communication may comprise one or more selected ones of the identified clinical statements and/or the identified clinical statements may be ordered according to their respective scores (e.g. higher to lower scores).

Alternatively or in addition, one or more identified clinical statements may be structured into sections according to the respective scores assigned to the one or more identified clinical statements, moved to a different section according to the respective scores assigned to the one or more identified clinical statements, and/or duplicated according to the respective scores assigned to the one or more identified clinical statements (e.g. if the score indicates that one or more identified clinical statements are important). In some embodiments, the generated record may comprise an indication of the participant that made the one or more identified clinical statements. For example, each of the one or more identified clinical statements may be annotated or tagged with the participant that mentioned that identified clinical statement. The indication of the participant may comprise an indication of the identity of the participant, such as their name.

Although not illustrated in Fig. 2, in some embodiments, the one or more processors 102 of the apparatus 100 may be configured to control at least one user interface 106 to render (e.g. output, display or provide) the generated record of the verbal communication. As mentioned earlier, the apparatus 100 may comprise at least one such user interface 106 and/or at least one such user interface 106 may be external to (e.g. separate to or remote from) the apparatus 100. In some embodiments, the generated record of the verbal communication may be rendered in real time. For example, one or more identified clinical statements may be added to the record of the verbal communication in real time. Where the verbal communication is during a meeting, the generated record of the verbal communication may be rendered in real time during the meeting, e.g. in the same room as the participants.

In some embodiments, the at least one user interface 106 may follow the layout of various steps of the meeting and add the one or more clinical statements to the step to which they relate. In some embodiments, the generated record of the verbal communication or, more specifically, one or more clinical statements of the generated record of the verbal communication may be editable. In this way, for example, participants can manually update one or more of the clinical statements of the generated record of the verbal communication. In some embodiments, the one or more processors 102 of the apparatus 100 may be configured to identify edits to the verbal communication (or one or more clinical statements of the verbal communication) and use this information as feedback to improve a model on which to base the processing of future verbal communications.

Although also not illustrated in Fig. 2, in some embodiments, the one or more processors 102 of the apparatus 100 may be configured to determine whether there is a discrepancy (e.g. a difference and/or incompatibility) between the content of two or more identified clinical statements. In this way, for example, clinical statements that may need additional review can be highlighted. In some embodiments, the one or more processors 102 of the apparatus 100 may be configured to compare the content of two or more identified clinical statements in order to determine whether there is a discrepancy between the content.

In some embodiments involving a comparison of the content of two or more identified clinical statements, the one or more processors 102 of the apparatus 100 may be configured to assign the score to a clinical statement at block 206 of Fig. 2 based on the content of the clinical statement, the expertise of the participant that made the clinical statement, and an output of the comparison.

Alternatively or in addition, in some embodiments, the one or more processors 102 of the apparatus 100 may be configured to, for each of the one or more identified clinical statements, compare the content of the clinical statement to a content of one or more reference clinical statements stored in at least one memory 104 and/or medical data stored in at least one memory 104. The one or more reference clinical statements may, for example, comprise one or more previous clinical statements, such as from previous (e.g. similar) cases, and/or may be based on clinical literature. As mentioned earlier, the apparatus 100 may comprise at least one such memory 104 and/or at least one such memory 104 may be external to (e.g. separate to or remote from) the apparatus 100. In some of these embodiments, the one or more processors 102 of the apparatus 100 may be configured to, for each of the one or more identified clinical statements, determine whether there is a discrepancy (e.g. a difference and/or incompatibility) between the content of the identified clinical statement and the content of the one or more reference clinical statements stored in at least one memory 104 and/or the medical data stored in at least one memory 104.

In some embodiments, the determination of whether there is a discrepancy between the content of the identified clinical statement and the content of the one or more reference clinical statements stored in at least one memory 104 may be based on language processing. For example, the determination may involve checking whether the content of the identified clinical statement is in line (e.g. consistent) with the content of the one or more reference clinical statements stored in at least one memory 104, e.g. in terms of medical conclusion and/or treatment steps. A discrepancy may be determined if the content of the identified clinical statement is in not in line (e.g. is inconsistent) with the content of the one or more reference clinical statements or if there is a predefined amount of content of the identified clinical statement that is in not in line (e.g. inconsistent) with the content of the one or more reference clinical statements.

In some embodiments, the medical data stored in at least one memory 104 can comprise medical data for a subject (e.g. patient) to which the verbal communication relates and/or medical data associated with clinical guidelines. The medical data for a subject (e.g. patient) to which the verbal communication relates can, for example, comprise subject (e.g. patient) data, such as facts and/or properties derived from a subject (e.g. patient) record. For example, the clinical statement "This form of cancer does not be seem to run in the subject's family" may be checked against subject data to determine whether this is indeed the case. If there is no evidence found from the subject data that one or more relatives of the subject have been diagnosed with cancer or a similar disease, a discrepancy may be determined. The medical data associated with clinical guidelines can, for example, comprise procedure data, such as a structured (meeting) agenda comprising one or more steps that the participants of the verbal communication need to follow (e.g. to arrive at a treatment selection). Thus, the one or more clinical statements can be checked against multiple data sources.

In some embodiments involving a comparison of the content of the clinical statement to a content of one or more reference clinical statements and/or medical data, the one or more processors 102 of the apparatus 100 may be configured to assign the score to a clinical statement based on the content of the clinical statement, the expertise of the participant that made the clinical statement, and an output of the comparison. For example, if a discrepancy is determined as a result of the comparison, the one or more processors 102 of the apparatus 100 may be configured to assign a lower score to the clinical statement than if no discrepancy is determined as a result of the comparison. In some embodiments, the score may be lowered by a predefined factor X where a discrepancy is determined. In some embodiments, any one or more of the content of the clinical statement, the expertise of the participant that made the clinical statement and the output of the comparison may have a different weight when assigning the score to a clinical statement. That is, any one or more of the content of the clinical statement, the expertise of the participant that made the clinical statement and the output of the comparison may have a greater influence on the score that is assigned to the clinical statement. In some embodiments, the score assigned to a clinical statement may be used to select and/or prioritize the clinical statement.

In some embodiments involving a discrepancy determination, the one or more processors 102 of the apparatus 100 may be configured to generate an indication of a discrepancy if there is determined to be a discrepancy between the content of two or more identified clinical statements, between the content of one or more identified clinical statements and the content of one or more reference clinical statements stored in at least one memory 104, and/or between the content of one or more identified clinical statement and the medical data stored in at least one memory 104. In some embodiments, the one or more processors 102 of the apparatus 100 may be configured to control at least one user interface 106 to highlight the discrepancy (e.g. a part of one or more clinical statements in respect of which the discrepancy is determined) and/or to output a request for input (e.g. additional information and/or a manual review). As mentioned earlier, the apparatus 100 may comprise at least one such user interface 106 and/or at least one such user interface 106 may be external to (e.g. separate to or remote from) the apparatus 100. In this way, the discrepancy can be flagged.

In some embodiments involving a discrepancy determination, the generated record of the verbal communication may comprise the generated indication of the discrepancy. In some embodiments, the one or more processors 102 of the apparatus 100 may be configured to suggest a re-evaluation of a topic (or case) to which the verbal communication is directed, based on the discrepancy. In some embodiments involving a re-evaluation, the one or more processors 102 of the apparatus 100 can be configured to learn from an outcome of the re-evaluation in order to further enhance the method 200 for (e.g. similar) topics (or cases) in the future. A machine learning algorithm can be used for this purpose and a person skilled in the art will be aware of such algorithms.

In some embodiments, the one or more processors 102 of the apparatus 100 may be configured to analyze the one or more identified clinical statements to determine whether there is a clinical statement made by a particular participant and, if not, generate an indication that a clinical statement by the particular participant is missing. For example, the one or more processors 102 of the apparatus 100 can be configured to detect that a certain participant (e.g. specialist) did not make a clinical statement (e.g. did not comment on) a particular (e.g. important) aspect, and this may be flagged during the verbal communication by way of the indication according to some embodiments. The determination of whether there is a clinical statement made by a particular participant can be based on the medical data (e.g. subject data, such as patient data) mentioned earlier. For example, a prediction may be made that a clinical statement is expected to be made by a particular participant based on the medical data. For example, a clinical statement may be expected from a clinical geneticist if it is determined from the medical data that the subject to which the verbal communication relates is suffering from a disease that runs in their family and is related to genomics.

The indication that a clinical statement by the particular participant is missing may, for example, be rendered by at least one user interface 106. As mentioned earlier, the apparatus 100 may comprise at least one such user interface 106 and/or at least one such user interface 106 may be external to (e.g. separate to or remote from) the apparatus 100. The indication may be rendered at the end of the verbal communication, e.g. at the end of a meeting in which the verbal communication is taking place. In some embodiments, sections of the record of the verbal communication may be flagged that are missing input from certain participants in order for a predefined protocol to be followed.

Although not illustrated in Fig. 2, in some embodiments, the one or more processors 102 of the apparatus 100 may be configured to compare the content of the one or more identified clinical statements to an indication of the content that the verbal communication is to cover and determine whether any content that the verbal communication is to cover is missing from the content of the one or more identified clinical statements. In some of these embodiments, the one or more processors 102 of the apparatus 100 may be configured to, if content is determined to be missing, generate an indication that the content is missing.

In some embodiments, the one or more processors 102 of the apparatus 100 may be configured to generate a clinical decision based on the generated record of the verbal communication. In some of these embodiments, the one or more processors 102 of the apparatus 100 may be configured to control at least one user interface 106 (such as those mentioned earlier) to render (e.g. output, display or provide) the generated clinical decision. In some embodiments, the one or more processors 102 of the apparatus 100 may be configured to identify one or more participants that made one or more clinical statements that resulted or contributed in the generated clinical decision. Thus, there is provided an intelligent agent that can derive conclusions from the recorded data.

An example of the apparatus 100 described herein in use will now be described. In the example, a patient case is discussed during a multi-disciplinary (MDT) meeting. A chairperson opens (e.g. in a picture archiving and communication system, PACS, system) radiology images of the patient on a first (e.g. left) screen located in the room and opens a MDT application on a second (e.g. center) screen. The chairperson introduces the patient, and refers to the primary radiologist in the room for the radiologist to announce his findings in the images verbally. The radiologist mentions that he found two tumors in the images that are shown. Meanwhile, in the background, the apparatus 100 described earlier with reference to Figure 1 is performing (e.g. in real time) the method described earlier with reference to Figure 2.

In particular, the one or more processors 102 of the apparatus 100 analyze the verbal communication to identify one or more clinical statements made by one or more participants of the verbal communication. The one or more processors 102 of the apparatus 100 identify the clinical statement made by the radiologist, which is that the radiologist found two tumors in the images. The one or more processors 102 of the apparatus 100 may restructure the clinical statement according to some embodiments. For example, the clinical statement may be restructured as "two potential tumors in left lung".

The one or more processors 102 of the apparatus 100 identify the participant that made the clinical statement and the expertise of that participant in the manner described earlier. That is, the one or more processors 102 of the apparatus 100 identify that the radiologist made the clinical statement and identify the expertise of the radiologist (e.g. from a record of accomplishment for the specific radiologist). In the manner described earlier, the one or more processors 102 of the apparatus 100 assign a score to the clinical statement made by the radiologist based on a content of the clinical statement (e.g. compared to previous patient cases) and the expertise of the radiologist. In this example, a score of 89% is assigned to the clinical statement made by the radiologist.

In the manner described earlier, the one or more processors 102 of the apparatus 100 generate a record of the verbal communication, which comprises one or more identified clinical statements and the respective scores assigned to the one or more identified clinical statements. Thus, in this example, the one or more processors 102 of the apparatus 100 generate a record of the verbal communication, which comprises the clinical statement made by the radiologist and the respective score assigned to the clinical statement. For example, the record may comprise the note "two potential tumors in left lung" with a score of 89% next to the note.

Another participant of the verbal communication, a junior radiologist, does not agree with the clinical statement made by the radiologist and mentions that he has "doubts about a third tumor showing in the images". He refers to a previous patient case where he saw a similar pattern. In the same manner as that described earlier, the one or more processors 102 of the apparatus 100 identify this clinical statement made by the junior radiologist, identify that it was the junior radiologist that made the clinical statement, identify the expertise of the junior radiologist, and assign a score to the clinical statement made by the junior radiologist. The generated record of the verbal communication is updated to include the clinical statement made by the junior radiologist together with the score assigned to that clinical statement. In this example, the one or more processors 102 of the apparatus 100 check the clinical statement made by the junior radiologist against the images themselves and the referred patient case. The one or more processors 102 of the apparatus 100 identify that the junior radiologist is less experienced in the field and also that the junior radiologist has minimal experience of cancer detection. Thus, the score that is assigned to the clinical statement made by the junior radiologist may be much lower, e.g. 34%. The one or more processors 102 of the apparatus 100 also retrieve the previous patient case and compares the clinical statements (and the outcome of the patient treatment) to the current case.

In the generated record of the verbal communication, the one or more processors 102 of the apparatus 100 create a note with the clinical statement made by the junior radiologist and annotate the note with the score assigned to the clinical statement (which is indicative of the experience level of the junior radiologist), together with the outcome of the comparison to the previous patient case. The one or more processors 102 of the apparatus 100 determine that there is a discrepancy between the content of the identified clinical statement of the radiologist and the identified clinical statement of the junior radiologist and thus generate an indication of the discrepancy. The indication recommends a re-evaluation of the case, based on discrepancy.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved apparatus 100, method 200, and computer program product for generating a record of a verbal communication, which addresses the limitations associated with the existing techniques. The improved apparatus 100, method 200, and computer program product described herein can be useful in a variety of applications and, in particular, in verbal communications between participants of different disciplines and/or different levels of expertise. For example, the improved apparatus 100, method 200, and computer program product described herein can be useful in multi-disciplinary meetings, such as multi-disciplinary tumor board meetings.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (100) for generating a record of a verbal communication, the apparatus (100) comprising:
one or more processors (102) configured to:
analyze a verbal communication to identify one or more clinical statements made by one or more participants of the verbal communication;
for each of the one or more identified clinical statements:
identify the participant that made the clinical statement and the expertise of the participant that made the clinical statement; and
assign a score to the clinical statement based on a content of the clinical statement and the expertise of the participant that made the clinical statement; and
generate a record of the verbal communication, wherein the generated record comprises the one or more identified clinical statements and the respective scores assigned to the one or more identified clinical statements.

2. The apparatus (100) as claimed in claim 1, wherein:
the generated record comprises the one or more identified clinical statements organized according to the respective scores assigned to the one or more identified clinical statements.

3. The apparatus (100) as claimed in any of the preceding claims, wherein:
the one or more processors (102) are configured to:
assign the score to the clinical statement based on the content of the clinical statement and whether the participant that made the clinical statement has expertise in a field to which the content of the clinical statement relates.

4. The apparatus (100) as claimed in any of the preceding claims, wherein:
the higher the expertise of the participant that made the clinical statement, the higher the score that is assigned to the clinical statement.

5. The apparatus (100) as claimed in any of the preceding claims, wherein:
the one or more processors (102) are configured to:
for each of the one or more identified clinical statements:
compare the content of the clinical statement to a content of one or more reference clinical statements stored in at least one memory (104) and/or medical data stored in at least one memory (104).

6. The apparatus (100) as claimed in claim 5, wherein:
the medical data stored in the at least one memory (104) comprises medical data for a subject to which the verbal communication relates and/or medical data associated with clinical guidelines.

7. The apparatus (100) as claimed in claim 5 or 6, wherein:
the one or more processors (102) are configured to:
assign the score to the clinical statement based on the content of the clinical statement, the expertise of the participant that made the clinical statement, and an output of the comparison.

8. The apparatus (100) as claimed in any of the preceding claims, wherein:
the one or more processors (102) are configured to:
determine whether there is a discrepancy between the content of two or more identified clinical statements; and
if there is determined to be a discrepancy between the content of two or more identified clinical statements, generate an indication of the discrepancy.

9. The apparatus (100) as claimed in claim 8, wherein:
the generated record of the verbal communication comprises the generated indication of the discrepancy.

10. The apparatus (100) as claimed in any of the preceding claims, wherein:
the one or more processors (102) are configured to:
control at least one user interface (106) to render the generated record of the verbal communication.

11. The apparatus (100) as claimed in any of the preceding claims, wherein:
the generated record comprises an indication of the participant that made the one or more identified clinical statements.

12. The apparatus (100) as claimed in any of the preceding claims, wherein:
the one or more processors (102) are configured to:
compare the content of the one or more identified clinical statements to an indication of the content that the verbal communication is to cover;
determine whether any content that the verbal communication is to cover is missing from the content of the one or more identified clinical statements; and
if content is determined to be missing, generate an indication that the content is missing.

13. The apparatus (100) as claimed in any of the preceding claims, wherein:
the verbal communication comprises a real-time verbal communication and/or a recorded verbal communication.

14. A computer-implemented method (200) for generating a record of a verbal communication, the method comprising:
analyzing (202) a verbal communication to identify one or more clinical statements made by one or more participants of the verbal communication;
for each of the one or more identified clinical statements:
identifying (204) the participant that made the clinical statement and the expertise of the participant that made the clinical statement; and
assigning (206) a score to the clinical statement based on a content of the clinical statement and the expertise of the participant that made the clinical statement; and
generating (208) a record of the verbal communication, wherein the generated record comprises the one or more identified clinical statements and the respective scores assigned to the one or more identified clinical statements.

15. A computer program product comprising a computer readable medium, the computer readable medium having a computer readable code embodied therein, the computer readable code being configured such that, on execution by a suitable computer or processor, the computer or processor is caused to perform the method as claimed in claim 14.
